Europäisches Patentamt

European Patent Office

Office européen des brevets

(11) Publication number: **0 172 178 B1**

(12) **EUROPEAN PATENT SPECIFICATION**

(45) Date of publication of patent specification: **23.10.91**

(51) Int. Cl.5: **C12N 11/08**, C12N 11/10, C12N 11/14, A61K 9/38, A61K 9/42, //C12N5/00, C12N1/20,(C12N11/08, C12R1:01),(C12N11/10, C12R1:01),(C12N11/14, C12R1:01)

(21) Application number: **85900598.5**

(22) Date of filing: **06.02.85**

(86) International application number: **PCT/AU85/00018**

(87) International publication number: **WO 85/03520 (15.08.85 85/18)**

(54) **METHOD FOR CELL CULTURE.**

(30) Priority: **06.02.84 AU 3477/84**

(43) Date of publication of application:
**26.02.86 Bulletin 86/09**

(45) Publication of the grant of the patent:
**23.10.91 Bulletin 91/43**

(84) Designated Contracting States:
**AT BE CH DE FR GB LI LU NL SE**

(56) References cited:
| | |
|---|---|
| EP-A- 15 473 | EP-A- 0 184 640 |
| WO-A-83/02954 | AU-B- 3 049 177 |
| DE-A- 1 617 501 | GB-A- 1 478 272 |
| GB-A- 2 093 040 | US-A- 4 055 466 |
| US-A- 4 237 033 | US-A- 4 415 668 |
| US-A- 4 448 884 | |

R. Baserga: "Tissue growth factors", 1981; Springer-Verlag, Berlin, DE .G. HAM: Survival and growth requirements of nontransformed cells.

(73) Proprietor: **SURFACE CONCEPTS PTY. LTD.**
**196 Belmont Street**
**Alexandria, NSW 2015(AU)**

(72) Inventor: **KJELLEBERG, Staffan**
**Skytteskogsgatan 6**
**S-414 76 Goteborg(SE)**
Inventor: **KEFFORD, Bruce**
**22A Essex Road**
**Surrey Hills, VIC 3127(AU)**

(74) Representative: **Lautmann, Kurt O.**
**KURT LAUTMANNS PATENTBYRA AB Box 245**
**S-691 25 Karlskoga(SE)**

## Description

The present invention relates to:

1) processes for the surface culture of eukaryotic cells and to substances thereby otained

2) processes for improved comfort and healing of skin lesions.

It is known that anchorage dependent (eukaryotic) cells can only be successfully cultured by the surface culture method. Thus eukaryotic cells held in suspension do not grow to the same extent. If however, eukaryotic cells are allowed to settle on a suitable surface, they can multiply up to contact inhibition.

The present invention utilizes a process which comprises the use of suitable beads made of, for example, dextran, or other material of inert matter such as plastics, glass and ceramics,of suitable diameter e.g. between 0.15 and 0.25 mm, suspended in a culture medium and held in suspension by stirring. Cells can be cultured on the surface of these beads using suitable techniques (see Bio-technology and Bio-engineering, volume XXI, 433-442 (1979 ). The available surface area given by the medium volume relation is especially large in the case of this process. Thus, the surface area of 3,000 cm$^2$ is available with 0.5 gms of dextran carriers in 100 mls of medium. Whilst the use of suitable beads is preferred any solid surface, preferably with a large surface area to volum ratio, can be used, for example fibres, tubing sheets, and particles.

Historically all tissue culture systems have required either serum or albumin components for growth. The regulation of growth and differentiation in mammalian cells is a complex network of external stimuli, mediated by both cognate cellular interactions and soluble signal factors produced by differentiating cells. To reproduce the growth requirements of normal mammalian cells in in vitro cultures is consequently a complex and difficult endeavor, which is far from being solved. The in vitro culture of continous cell lines, with transformed growth requirements and characteristics, have advanced to the present point where a synthetically composed culture medium consisting of a mixture of salts, hormones, vitamins, and amino acid still has to be supplemented with a oxygenically derived serum. This serum supplement is necessary to achieve an optimal cell growth and contains a variety of known as well as unknown fatty acids, hormones, metal ions, proteins, and carbohydrates etc. It is conceivable that a mammalian cell could construct all of its lipids, carbo-hydrates and proteins from amino acids, numerous vitamins and glucose etc. however this is proved exceedingly difficult to prove correct. It is also noteworthy that in mammalian tissues the fatty acids content is generally a reflection of the diet and the dietary intake of f.a. and that the f.a. are taken up with little modification (and incorporated as phospholipids and triglycerides). It is evident that f.a. are important building blocks for cells and that it is essential to provide long chain f.a. for normal cell growth. Further evidence that cells in tissue culture highly value long chain f.a. is the rapid uptake and gorging that occurs when fatty acids are supplied.

Cells can be grown in the absence of serum components provided long chain f.a. are supplied. It would seem that this is the main function of serum components although there are reports that suggest that cell adhesion factors and some hormones are important for the growth of some cells.

The problem with supplying cells with long chain fatty acids in the absence of albumin is that the fatty acids at the concentrations required for growth would be toxic unless bound to a solid surface. Use of phospholipid vesicles for instance seem to cause a toxicity problem.

It is clear that cells in culture can modify the fatty acids supplied either by desaturation, shortening or elongation of the molecules. It is possible to provide long chain fatty acids localised on a solid surface suitable for the growth of tissure culture cells. Fatty acids supplied in this way remove the requirement of soluble fatty acids and therefore would remove or greatly reduce the requirement of serum fractions.

Existing media used for tissue cell culturing,including micro carrier cultivation of eucaryotic cells, suffer from a number of disadvantages.

They are extremely costly, as well as the product from the culture growth is undefined and the quality of the culture growth cannot be standardized and varies from batch to batch. Furthermore the risk of contamination is high and the preparation of the final product involves tedious and laborious work. Additionally, there is a diminishing world supply of foetal calf serum. For example in the large scale cell cultures of hybridoma cells, used in the preparation of monoclonal antibodies, the use of foetal calfserum has become impossible due to the disadvantages mentioned above. Much research is today being done to develop an albumin free media. The Tween fraction of the albumin-Tween complex is purified; an expensive procedure, which because of its expense is only used for media in the production of vaccines.

The advantages of introducing simplifications of the media such as the present invention are:

i) a defined medium

ii) a medium that can be manipulated in terms of the f.a. provided for growth

2

iii) considerable reduction in cost

iv) supply of lipids that are natural, as components of cell lipids are different from serum lipids.

The following pieces of information, related to the role of polar lipids and fatty acids as well as tissue cultur growth on surfaces, are obtained from the literature. The function of serum components can be replaced by fatty acids (continuous infusion) (Lynch and Liffmann 1981; Lynch 1980). Serum albumin is strictly needed as a carrier for f.a. and was shown to have no other stimulatory growth effects (Lynch and Liffmann; 1981).

Unsaturated f.a. bound to BSA enchance the cellular growth and membrane fluidity of cultured cells (Yamane et al. 1981).

Similar growth rates are obtained with f.a. infusion (continuous) as with albumin f.a. complex (Lynch and Liffmann; 1981).

Palmitic acid, stearic acid and oleic acid make up ca. 70% of the total f.a. in the cell. F.a.composition of the L-cells probably reflects the fatty acid composition of the foetal bovine serum in which they are grown. However the cells are able to desaturate, elongate and shorten f.a. (Weinstein et al 1969)

Bailey et al. (1964) have shown that culture mammalian cells derive most of their lipids required for growth from the lipids of the medium and Bailey (1967) that lipid synthesis is inhibited up to 95% in the presence of serum lipids.

Phospholipids may be useful for feeding cells (Pagano et al. 1974). Human diploid fibroblast cells, (used for e.g. interferon production), have been grown at a slower rate in the absence of serum indicating that it is likely that these cells will be suitable for our purposes. It is probable that the reduced growth rate is due to the absence of exogenous fatty acids.

It has been shown that the net negative charge of surfaces is suitable for tissue culture growth (Davies 1981) Fatty acid coated surfaces have a net negative charge.

The present invention is based on the discovery that the above-mentioned disadvantages in the surface culture method, especially in the production of cell culture producible substances such as interferon, viruses, enzymes, antibodies and recombinant DNA production, may be overcome by effecting the cell culture according to the surface culture method in a media free of foetal calf serum and/or albumin/fatty acids, or with very reduced amounts of these.

Thus according to one feature of the present invention there is provided a process for the surface culture of eukaryotic cells which comprises culturing the said cells on a solid carrier in a media free of foetal calf serum.

The present invention is based on coating surface active molecules, which have a very low solubility in an aqueous solution, onto a solid surface. Surface active molecules are held in position at the solid surface by hydrophobic forces, in defined structures made up by closely packed monolayers. There is no bond between the molecules and the surface and the process can be considered as an immobilization of the surface active molecules.

The growth cells can, using the present invention, be cultured on or in the precense of a solid carrier coated with surface active molecules which act as a major carbon- and energy source. The aqueous medium can be any convenient medium without foetal calf serum or albumin - compounds, or with reduced amounts of these.

The advantages of the present invention are numerous. The system is defined, has a low cost of production and is easy to prepare and handle.

The present invention is a totally new concept of feeding culture cells and the method of the present invention can be used for the production of protein free vaccine and it is possible to use any surface active material or use any solid surface for cell culture growth. The diluents used can be any aqueous media and organic solvent. The washing procedure can be by delution, chromatography, filtering, centrifugation and re-suspension.

The process according to the invention simply involves making the surface active molecule soluble in the solvent and then exposing this mixture to the solid surface and later washing away any unbound surface active molecules.

Examples of surface active molecules include phospholipid, triglyceride, any fatty acid, any protein, synthetic peptide, hydrocarbon and sterol.

Examples of a solvent include any aqueous medium, organic solvent, for example chloroform, acetone or hydrocarbons.

Examples of solid surfaces include beads, fibres, tubing sheets and particles.

The washing procedure simply involves exposing the coated surface to large volumes of solvent (usually water) to allow the unbound surface active molecules to be removed.

A non-limiting example illustrates the bases of the process forming the grounds of the present invention:

100 ml Octyl-Sepharose beads in suspension is pelleted (i.e. centrifuged at 300 g for 2 min.) and resuspended in 100 ml of acetone (repeat). The suspension is pelleted again plus resuspended in 100 ml of chloroform (repeat). 1 mg of stearic acid is dissolved in 10 ml of chloroform and added and stirred for 10 min. The solvent is evaporated to dryness and 100 ml of acetone is added, stirred and evaporated to dryness (repeat). Beads are resuspended in 1 litre of distilled water and allowed to settle and the supernatant liquid is removed by vacuum suction with particular care being taken to remove fatty acids accumulated at the water surface (repeat eight times). After the final wash, the beads are allowed to settle and the bulk of the water is removed. The suspension is autoclaved at 15 psi for 15 min and allowed to cool. The beads are washed twice in 120 ml of sterile medium used in culturing. The beads are dispensed into a further volume of medium for culturing. The beads are used as supports or combination with other beads for the culturing of cells.

The invention is further illustrated with reference to the following non-limiting examples of experiments using the princple of the invention for growth of tissue culture cells and Leptospira.

Cells

3T3 and Vero

The cells were trypsinated off the "mother" flask with 0,25% trypsin 5-10 min at 37°C. After centrifugation for 5 min. at 1000 x g in medium containing 10% FCS,the cells were washed three times and resuspended in serum free DME. Inoculation: 5 x 10⁵ cells per P3 dish.

Media

Basal medium,DME/F10 (80:20, v/v) was supplemented with 2 mM glutamine, 1% non-essential aminoacids.
Control medium: Basal medium with 10% FCS.
ITAF: Basal medium with
1 μg Insulin
25 μg Transferrin
1 mg Albumin
2 μg Fibronectin
added per ml medium FIT: Basal medium with
1 μg Fibronectin
1 μg Insulin
25 μg Transferrin
added per ml medium

Beads: Octyl - Cytodex 1 beads were coated with fatty acid according to the description above, but the last rinse was performed with 0,9% NaCl instead of distilled water.

Culturing and assay

Experiment were performed in 60 mm bacteriological p3 dishes (Nunc) with 0,5 ml cellsuspension and incubated over night at 37°C with humified atmosphere with 5% $CO_2$. In the morning 2 ml fresh medium was added. After 72 hours the cytodex suspension was removed and after sedimentation of beads the supernatant was sihponed off, 500 μl cristalviolett (Merck 1408) in citric acid was added. After 60 min incubation at 37°C 5% $CO_2$ the cell nuclei were counted in a Burgher-counter chamber.

Result and discussion

It was shown that fatty acid coated beads can not replace the addition of FCS to the medium. However using a defined medium a significant increase is observed with the coated beads (see table 1 & 2). In one experiment the cell yield was 89% of the control.

When the fatty acid is bound to the beads there were no toxicity problems with the fatty acid.

After coating with fatty acid the coated beads are both mechanically and heat stable and withstand autoclaving at 115°C for 15 min.

The cells to be attached see the characteristies of the coated beads i.e. the surface active material (fatty acid) and not the actual surface of the bead itself.

Gorging of fatty acids by attached cells was initially observed. This induced rounding up and less spreading out of the cells. This problem was however not encountered in any of the subsequent experiments. Should this problem be encountered it can be overcome by mixing non coated beads with coated where the non coated beads absorb the excess of fatty acid from the coated ones. This mechanism also acts as a detoxification of free fatty acid.

Cells fed on fatty acids were observed to use stores - reserve material of fatty acids. When preparing the coated beads better washing procedure has to be employed.

In another set of experiment performed with the microorganism Leptospira strain HARDJO similar results as mentioned above has bee obtained (see table 3). Leptospira utilize fatty acids in the same metabolic pathways as tissue culture cells do, and Leptospira requires long chain fatty acids for growth. The fatty acids are normally supplied as tween80 with albumine fraction v (1%) to bind any free, toxic fatty acid. When using fatty acid coated beads in a carbon free medium with 10 % tween80 albumine gave a growth yield of 60% of a fullstrength Leptospira medium after four days of growth (table 3).

| Table 1 | | B E A D S | |
|---------|--------|-------------------|----------------------------------|
| Cells | Medium | Octylcytodex 1 | Octyl - Cytodex 1 + linoleic acid |
| 3T3 | Control | 100 | 100 |
| | ITAF | 39 | 47 |
| | FIT | 0 | 0 |
| Vero | Control | 100 | 91 |
| | ITAF | 26 | 89 |
| | FIT | 0 | 26 |

Figures in table 1 are given as % of growth on Octyl-cytodex 1 with control medium.

5

| Table 2 | | B   E   A   D   S | |
|---|---|---|---|
| Cells | Medium | Octylcytodex 1 | Octyl - Cytodex 1 + linoleic acid |
| 3T3 | Control | 100 | 148 |
| | ITAF | 16 | 30 |
| | FIT | 7 | 68 |

Figures in table 2 are given as % of growth on Octyl-cytodex 1 with control medium
Tabel 2 presents the results of separate sets of experiment.

| Table 3 | No of cells per ml |
|---|---|
| Tween80 albumin medium (Full strength Leptospira medium) | $5.5 \times 10^8$ |
| Coated beads in carbon free basal medium + 10% Tween80 albumine | $3.3 \times 10^8$ |
| Carbon free basal medium without Tween80 albumine | $2.6 \times 10^7$ |
| Ino  culum concentration | $1.0 \times 10^7$ |

Results are expressed as numberof cells per ml after four days of growth of Leptospira strain HARDJO. A tenfold reduction of Tween80 albumine concentration gave a 60% growth yield of full strength Leptospira medium.

The effect of the invention can be further illustrated in two other growth experiments.

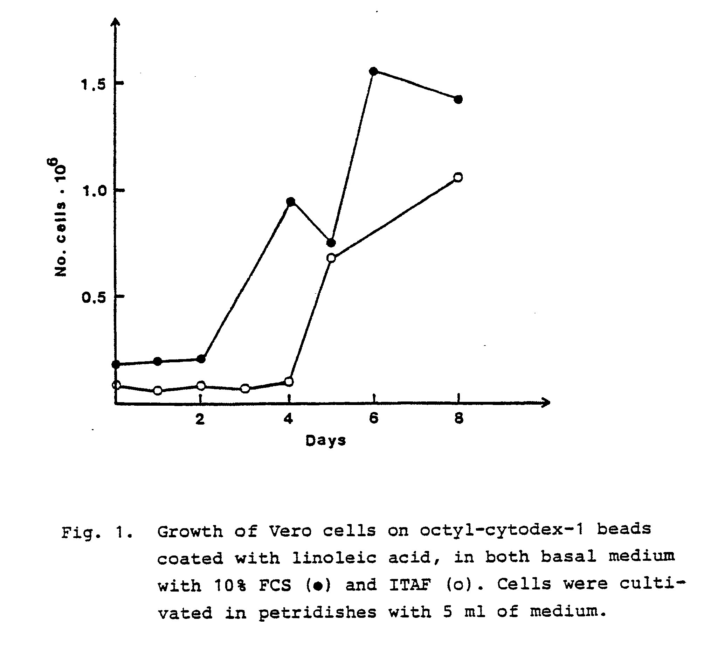

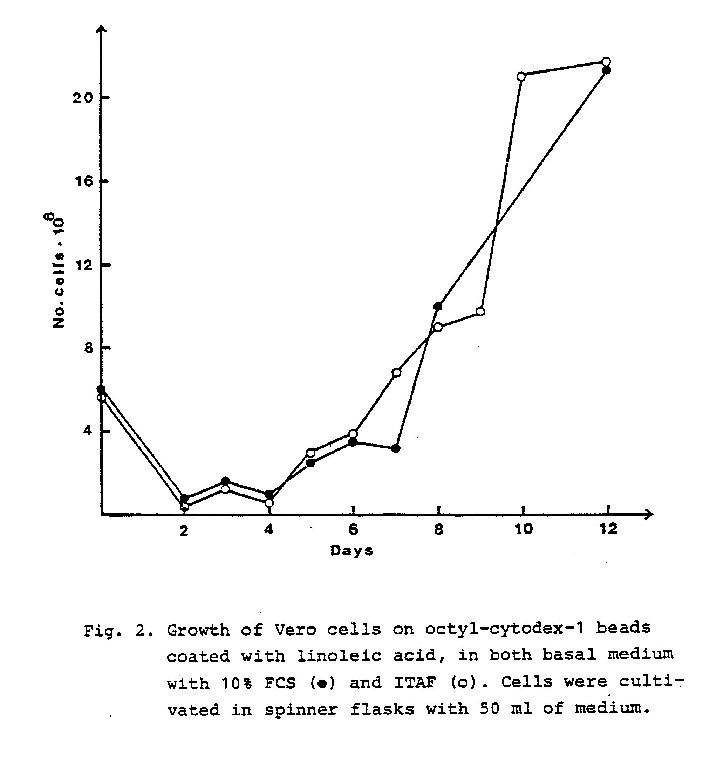

Vero cells grown on octyl-cytodex-1 beads with linoleic acid in both basal medium with 10% FCS and ITAF was performed in P3 dishes (Fig. 1) as well as in spinner flasks (Fig. 2) with 50 ml medium.

As can be seen from the two figures growth of vero cells in medium lacking 10% FCS was equally good as growth in medium containing 10% FCS.

The following application relates to processes for improved comfort and healing of skin lesions.

Currently sterilized and dried beads are used to absorb liquid from skin lesions. This dries the lesions and leads to improved comfort and healing. The advantage of this mentioned technique is that the entire internal surface of the lesion is covered and drained whereas normal adhesive bandages do not penetrate below the outer surface of the lesion and drainage is frequently poor. Further this above technique acts on a near cellular level as individual beads contact cells.

The present application is an adaption of the above technique based on two premises. (i) Some free fatty acids are toxic to bacteria and can be bacteriocidal and/or bacteriostatic. (ii) There is evidence that the provision of certain free fatty acids at the lesion may improve the rate of healing.

Clearly these two factors would lead to improved healing and reduced infection.

The process according to the application simply involves making the surface active molecule soluble in the solvent and then exposing this mixture to the solid surface and later washing away any unbound surface active molecules. Examples of surface active molecules, solvents and solid surfaces are given above (page 6 ). The washing procedure simply involves exposing the coated surface to large volumes of solvent (usually water) to allow the unbound surface active molecules to be removed. The following method is an example of the process according to the present invention illustrating a further application, 100 ml Octyl-Sepharose beads in suspension is pelleted (i.e. centrifuged at 300 g for 2 min.) and resuspended in 100 ml of acetone (repeat). The suspension is pelleted again and resuspended in 100 ml of chloroform (repeat). 1 mg of stearic acid is dissolved in 10 ml of chloroform and added and stirred for 10min. The solvent is evaporated to dryness and 100 ml acetone is added, stirred and evaporated to dryness (repeat). Beads are resuspended in 1 litre of distilled water and allowed to settle and the supernatant liquid is removed by vacuum suction with particular care being taken to remove fatty acids accumulated at the water surface (repeat eight times). After the final wash, the beads are allowed to settle and the bulk of the water is removed. The coated beads are heat sterilized until complete dryness.

In closing, it is to be noted that the preceding detailed description is, in the main, merely illustrative of the invention - and hence should not be limitatively construed. Clearly to those skilled in the art, variation in technique and /or non critical details will suggest themselves,however such non-essential features lie within the ambit of the present invention.

## Claims

1. Method for culture of eucaryotic and procaryotic cells on surfaces,
   **characterized** in, that the culture is performed in media with reduced amounts of, or free of, foetal calf serum, using surfaces activated through coating with surface active molecules such as phospholipids, triglycerids, fatty acids, sterols, surface active proteins and synthetic peptides.

2. Method according to claim 1,
   **characterized** in using surface active molecules having very low solubility in aqueous solution and being held in position at the solid surface by hydrophobic forces in defined structures made up by closely packed monolayers.

3. Method according to claims 1 - 2,
   **characterized** in that the surface active molecules act as a major carbon- and energy source.

4. Method according to claims1 - 3,
   **characterized** in making the surface active molecule soluble in a solvent and then exposing the mixture to a solid surface and later washing away any unbound surface active molecules.

5. Products for healing skin leasions,
   **characterized** in, that they are made using the procedure according to claims 1 - 4.

6. Products for healing skin leasions according to claim 5,
   **characterized** in that they are in the form of powders, ointments, solutions or emulsions.

## Revendications

1. Méthode pour la culture en surfaces de cellules encaryotiques et procaryotiques,
   **caractérisée** en ce que la culture est réalizée dans un milieu ayant une faible teneur en, ou libre de, serum foetal de veau, utilisant les surfaces activées par revêtement de molécules á surface active, telles que les phospholipides, les triglycérides, les acides gras, les stérols, les protéines á surfaces active et les peptides synthétiques.

2.  Méthode selon la revendication 1,
    **caractérisée** par l'utilisation de molécules á surface active ayant une très faible solubilité en solution aqueuse et maintenues en place sur la surface active par des forces hydrophotiques en structures défines constituées par des monocouches fortement serrées.

3.  Méthode selon les revendications 1 - 2,
    **caractérisée** en ce que les molécules á surface active agissent comme une source importante de carbone et d'énérgie.

4.  Méthode selon les revendications 1 - 3,
    **caractérisée** en rendant la molécule á surface active soluble dans un solvant et en déposant ensuite le mélange sur une surface solide pins en éliminant par lavage toute molécule á surface active non fixée.

5.  Produits pour la cicatrisation de lésions cutanées,
    **caractérisés** en ce qu'ils sont produits en utilisant la méthode selon les revendications 1 - 4.

6.  Produits pour la cicatrisation de lésions cutanées selon la revendication 5, **caractérisés** en ce qu'ils existent sous forme de poudres, d'onguents, de solutions ou d'émulsions.

**Patentansprüche**

1.  Verfahren zum Züchten von eukaryotischen und prokaryotischen Zellen auf Oberflächen, dadurch **gekennzeichnet,** daß die Züchtung in Närböden mit reduzierten Mengen an, oder frei von fötalem Kalbsserum erfolgt, wobei man mit Oberflächen arbeitet, die durch einen Überzug mit oberflächenwirksamen Molekülen aktiviert werden, wie Phospholipide, dreiwertige Glyceride, Fettsäuren, Sterine oder oberflächenwirksame Proteine und synthetische Peptide.

2.  Verfahren nach Patentanspruch 1, dadurch **gekennzeichnet,** daß oberflächenwirksame Moleküle mit einer sehr niedrigen Löslichkeit in wäßrigen Lösungen benutzt und die an der festen Oberfläche in der richtigen Lage gehalten werden und zwar durch wasserabweisende Kräfte, die nach den beschriebenen Strukturen in dicht gepreßten Einzelschichten zusammengestellt sind.

3.  Verfahren nach den Patentansprüchen 1 - 2, dadurch **gekennzeichnet,** daß die oberflächenwirksamen Moleküle als Hauptkohlenstoff- und Energiequelle wirken.

4.  Verfahren nach den Patentansprüchen 1 - 3, dadurch **gekennzeichnet,** daß die oberflächenwirksamen Moleküle in einer Lösung löslich gemacht werden und dann das Gemisch auf die feste Oberfläche aufgetragen wird und später alle ungebundenen, oberflächenwirksamen Moleküle abgespült werden.

5.  Erzeugnisse zur Heilung von Hautleiden, dadurch **gekennzeichnet,** daß sie nach den unter Nutzung der in den Patentansprüchen 1 - 4 genannten Verfahren hergestellt werden.

6.  Erzeugnisse zum Heilen von Hautleiden nach Patentanspruch 5, dadurch **gekennzeichnet,** daß sie die Form von Puder, Salben, Lösungen oder Emulsionen haben.

Fig. 1. Growth of Vero cells on octyl-cytodex-1 beads coated with linoleic acid, in both basal medium with 10% FCS (●) and ITAF (o). Cells were culti- vated in petridishes with 5 ml of medium.

Fig. 2. Growth of Vero cells on octyl-cytodex-1 beads
coated with linoleic acid, in both basal medium
with 10% FCS (●) and ITAF (o). Cells were culti-
vated in spinner flasks with 50 ml of medium.